# NOUVEAU FASCICULE DE BREVET EUROPEEN

(11) **EP 0 293 579 B2**
(45) Date de publication et mention de la décision concernant l'opposition: **03.09.1997**
(45) Mention de la délivrance du brevet: 10.04.1991
(21) Numéro de dépôt: 88105810.1
(22) Date de dépôt: 12.04.1988
(51) Int. Cl.: A61K 7/42, A61K 7/48

(54) **Compositions cosmétiques contenant un complexe cuivrique de l'acide 3,5-diisopropyl salicylique pour la protection contre le rayonnement UV et utilisation d'un tel composé en cosmétique**
Kosmetisches Mittel zum Schutz gegen UV-Strahlung, das einen Kupferkomplex des 3,5-Diisopropyl-salicylsäure enthält sowie dessen Verwendung in Kosmetika
Cosmetic composition for protection against UV radiations containing a cupric complex of 3,5-diisopropyl-salicylic acid, and the cosmetic use of such a compound

(30) Priorité: 13.04.1987 LU 86844
(43) Date de publication de la demande: 07.12.1988
(73) Titulaire: L'OREAL, 75008 Paris (FR)
(72) Inventeur: Hocquaux, Michel, F-75012 Paris (FR); Rosenbaum, Georges, F-92600 Asnières (FR)
(74) Mandataire: Casalonga, Axel

(56) Documents cités:
- AU-A- 610 868
- US-A- 180 556
- US-A- 3 821 370
- US-A- 4 136 165
- US-A- 4 199 576
- US-A- 4 221 785
- US-A- 4 248 861
- CHEMICAL ABSTRACTS, vol. 105, 1986, page 378, résumé no. 48835q, Columbus, Ohio, US; & RO-A-87 799 (INTREPRINDEREA DE PRODUSE COSMETICE "MIRAJ") 30-12-1985
- Stedman's medical distionary, 24th ed. Mot : "erythema"
- Oxy radicals and their scavenger systems, vol. II, p. 173-178 and 208-209, Elsevier, New York, 1983
- Antitumor effect of a copper coordination compound with superoxide dismutase like activity, JNCL, Vol. 66/6 (June 1981), pp. 1077-1081
- Principles of internal medicine, 10th Ed., pp. 275-276 McGraw Hill, New York, mot : "Sunburn"
- Surburn cell formation is prevented by scavenging oxygen intermediates, CED, 8 pp. 305-310, Blackwell, 1983
- Role of oxygen radicals in tumor promotion, Environmental Mutagenesis 6, pp. 593-616, 1984
- Inhibition of ultraviolet light-induced erythema by antioxidants, Arcg. Derm. Res. 266, pp. 91-94, 1970
- Stedman's medical dictionary, 25th Ed., mots : "cosmetics", "medicament", "medicinal"
- Liste des substances que ne peuvent contenir les produits cosmétiques, Journal officiel des communautés européennes, pp. 174-183, 1976
- Merck Index, p. 1156 abs. 7248, p. 980 abs. 6141, p. 1254 abs. 7906 (quelques agents anti-inflammatoires)
- Déclaration du Docteur Kensler
- "Report of investigation of topical antiinflammatory properties of copper diisopropylsalicilate (CiDIPS)", 16/7/93, done pby Estée Lauder Inc.

## Description

### Description pour les Etats contractants suivants : BE, CH, DE, FR, GB, IT, LI, NL, SE, AT, GR

La présente invention est relative à une composition cosmétique contenant un complexe cuivrique de l'acide 3,5-diisopropyl salicylique.

Il est bien connu que la peau, soumise à l'irradiation solaire, subit différentes modifications et en particulier certains dommages.

Parmi les dommages causés à la peau par le soleil, le plus spectaculaire est incontestablement le coup de soleil ou èrythème solaire qui apparaît, selon l'intensité et la durée d'irradiation, entre la deuxième et la vingt quatrième heure après l'exposition au soleil. L'érythème solaire peut se manifester par un léger rosissement de la peau, mais peut aussi conduire à de véritables brûlures accompagnées de troubles importants de l'état général.

Le rayonnement solaire peut également provoquer des modifications cellulaires au niveau de la peau. On observe notamment la formation dans l'épiderme de kératinocytes dégradés, voire détruits par les radiations UV, qui sont des cellules dégénérées et nécrosées sous l'action des radiations solaires, généralement connues sous l'appellation de "sun burn cells"

Le domaine du rayonnement solaire responsable de ces phénomènes se situe dans les radiations ultraviolettes, c'est-à-dire dans la gamme de longueurs d'onde comprises entre 280 et 400 nm. On a donc cherché à protéger la peau par application de substances présentant un maximum d'absorption dans cette zone. Ces substances constituent ce que l'on appelle des filtres solaires. Selon la quantité de filtre solaire appliquée sur la peau, une partie plus ou moins importante des rayons nocifs est arrêtée, ce qui diminue d'autant l'apparition des dommages causés à la peau.

De nombreux filtres solaires ont déjà été préconisés, de natures chimiques diverses et présentant des caractéristiques physicochimiques variées, en particulier en ce qui concerne la solubilité (produits hydrosolubles ou liposolubles). Ces composés assurent ce que l'on peut appeler une protection primaire.

Cependant, l'absorption du rayonnement ultraviolet par les filtres n'est jamais totale. Il subsiste donc une quantité résiduelle de radiations énergétiques qui atteint la peau. Ainsi, les filtres solaires atténuent les dommages créés au niveau de la peau sans les supprimer totalement.

Il est donc souhaitable d'associer aux filtres solaires ou de les remplacer par des produits susceptibles de diminuer les dommages causés à la peau par un phénomène autre qu'une simple filtration.

La demanderesse a découvert que l'application sur la peau d'un complexe formé de deux molécules d'acide 3,5-diisopropyl salicylique et d'un atome de cuivre à savoir le bis(3,5-diisopropylsalicylate) de cuivre II, de formule 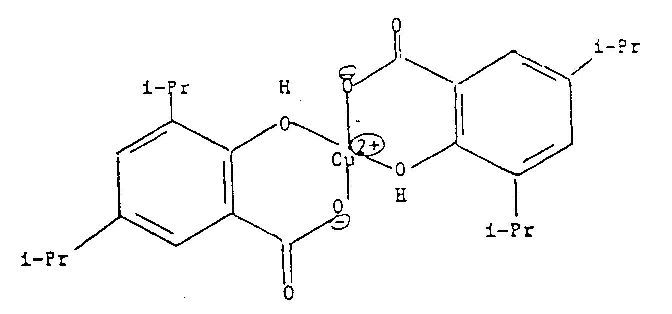 permettait de façon surprenante de réduire l'intensité de l'érythème solaire et le nombre de kérationocytes dégradés, sans que ces propriétés soient dues à un net pouvoir absorbant dans le domaine du rayonnement ultraviolet. Ces propriétés persistent en présence de filtres solaires.

Le complexe cuivrique de l'acide 3,5-diisopropyl salicylique est un composé connu dont la synthèse est décrite dans "Journal of Medicinal Chemistry", 1976, Vol. 19, n° 1, p 135-148. Ce document ainsi que le brevet US 4 221 785 décrivent également les propriétés anti-inflammatoires et anti-ulcère de ce complexe cuivrique pouvant être administré par voie parentérale, en particulier sous-cutanée, et par voie orale. La demande de brevet international WO 84/04922 décrit ce composé comme agent anti-tumoral administré par voie intramusculaire.

Cependant, l'application de ce complexe cuivrique par voie topique dans le domaine cosmétique, qui fait l'objet de la présente invention, n'est préconisée dans aucun des documents de l'art antérieur cités ci-dessus. Or, la demanderesse a pu démontrer que l'action de ce complexe cuivrique vis-à-vis des radiations UV s'exerce essentiellement à la surface de la peau.

La présente invention a pour objet une composition cosmétique utile pour la protection de la peau contre le rayonnement ultraviolet de longueurs d'onde comprises entre 290 et 400 nm comprenant, dans un support cosmétiquement acceptable contenant au moins une phase grasse, une quantité efficace de bis(3,5-diisopropylsalicylate) de cuivre II.

Un autre objet de l'invention est constitué par une composition cosmétique de protection de la peau comprenant, dans un support cosmétiquement acceptable contenant au moins une phase grasse, ledit complexe cuivrique de l'acide 3,5-diisopropyl salicylique et au moins un filtre solaire absorbant les rayons ultraviolets de longueurs d'onde comprises entre 280 et 400 nm.

D'autres objets et caractéristiques de l'invention apparaîtront à la lecture de la description qui va suivre.

La composition cosmétique selon l'invention contient, dans une phase grasse, le complexe cuivrique de l'acide 3,5-diisopropyl salicylique dans une gamme de concentrations allant de 0,01 à 5% et de préférence de 0,05 à 1% en poids, sur la base du poids total de la composition. Il est à noter que, bien que coloré en solution, le complexe cuivrique ci-dessus ne colore pas la peau aux concentrations utilisées.

En raison du caractère liposoluble du complexe cuivrique utilisé, les compositions cosmétiques selon l'invention contiennent au moins une phase grasse. Elles peuvent se présenter sous forme d'huiles, de lotions oléoalcooliques, d'émulsions telles que des crèmes ou des laits, de gels gras ou olécalcooliques, de bâtonnets solides ou être conditionnées en aérosol.

La phase grasse est constituée de corps gras tels que les huiles ou les cires minérales, animales ou végétales, les acides gras, les triglycérides d'acides gras ayant de 6 à 18 atomes de carbone et les alcools gras.

Outre la phase grasse, le support cosmétique peut aussi contenir des monoalcools ou polyalcools inférieurs contenant de 1 à 6 atomes de carbone ou bien de l'eau et des mélanges de ces différents constituants.

Les mono- ou polyalcools plus particulièrement préférés sont choisis parmi léthanol, l'isopropanol, le propylène glycol, le glycérol et le sorbitol. Les mélanges hydroalcooliques sont de préférence des mélanges d'eau et d'éthanol.

A titre de corps gras, on peut utiliser :
- des huiles minérales telles que l'huile de vaseline ;
- des huiles animales telles que les huiles de baleine, de phoque, de menhaden, de foie de flétan, de morue, de thon, de tortue, de suif, de pied de boeuf, de pied de cheval, de pied de mouton, de vison, de loutre, de marmotte ;
- des huiles végétales telles que des huiles d'amande, d'arachide, de germes de blé, d'olive, de germes de maîs, de jojoba, de sésame, de tournesol, de palme, de noix, de colza.

On peut également utiliser comme corps gras : la vaseline, la paraffine, la lanoline hydrogénée, la lanoline acétylée et les huiles de silicone.

On peut utiliser comme cires la cire de sipol, la cire de lanoline, la cire d'abeille, la cire de candellila, la cire microcristalline, la cire de camauba, le spermaceti, le beurre de cacao, le beurre de karité, les cires de silicones, les huiles hydrogénées concrètes à 25°C, les sucroglycérides, les oléates, myristates, linoléates et stéarates de Ca, Mg, Zn et Al.

Parmi les alcools gras, on peut citer les alcools laurique, cétylique, myristique, stéarique, palmitique et oléique.

La composition cosmétique selon l'invention peut également renfermer des émulsifiants non-ioniques, anioniques, cationiques ou amphotères.

Il peut aussi être avantageux d'utiliser des épaississants tels que les dérivés de cellulose, les dérivés d'acide polyacrylique, les gommes de guar ou de caroube, les gommes de Xanthane.

La composition cosmétique selon l'invention peut également contenir des adjuvants habituellement utilisés en cosmétique et notamment des produits hydratants, des adoucissants, des conservateurs, des colorants, des opacifiants, des parfums et des propulseurs ; les propulseurs sont des propulseurs classiques tels que les alcanes, les fluoroalcanes et les chlorofluoroalcanes.

La composition cosmétique selon l'invention possède un pH compris entre 4 et 9, et de préférence entre 5,5 et 8, qui peut éventuellement être ajusté à l'aide d'un agent de régulation du pH.

La composition cosmétique selon l'invention peut également contenir, en association avec le complexe cuivrique de l'acide 3,5-diisopropyl salicylique, au moins un filtre solaire absorbant les radiations de longueurs d'onde comprises entre 280 et 400 nm.

La concentration totale en filtre(s) solaire(s) est comprise entre 0,5 et 20% du poids total de la composition.

Ces filtres solaires sont choisis parmi les dérivés :
- de l'acide p-aminobenzoïque ;
- de l'acide cinnamique ;
- du benzylidène camphre ;
- de l'acide salicylique ;
- de l'acide anthranilique ;
- de l'acide urocanique ;
- de la benzophénone ;
- du dibenzoylméthane ;
- du benzotriazole ;
- de l'acide benzimidazole sulfonique.

Ils sont en particulier choisis parmi ceux cités dans l'article ; "UV absorbers in sun cosmetics 1978", D.H. LIEM and L.T.H. HILDERINK, International Journal of Cosmetic Science 1, 341-361 (1979).

On peut également utiliser des polymères filtres comme ceux décrits dans les brevets français 2 237 912 et 2 359 857 de la demanderesse.

Une forme de réalisation de l'invention est une émulsion sous forme de crème ou de lait comprenant, en plus du complexe cuivrique, des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation de la composition cosmétique de l'invention est constituée par des huiles à base d'huiles et cires naturelles ou synthétiques, de lanoline et d'esters d'acides gras, notamment de triglycérides d'acides gras, ou par des lotions oléoalcooliques à base d'huiles, de cires, d'esters d'acides gras tels que les triglycérides d'acides gras et d'alcools inférieurs tels que l'éthanol ou de glycols tels que le propylène glycol ou de polyols comme le glycérol.

La composition cosmétique de l'invention peut également être un gel gras comprenant des huiles ou des cires et un épaississant tel que la silice ; les gels oléoalcooliques comprennent en outre un ou plusieurs alcools ou polyols inférieurs tels que l'éthanol, le propylène glycol ou le glycérol.

Les bâtonnets solides sont constitués de cires et d'huiles naturelles ou synthétiques, d'alcools gras, d'esters d'acides gras, de lanoline et autres corps gras.

Les exemples suivants sont destinés à illustrer l'invention sans pour autant présenter un caractère limitatif.

### EXEMPLE 1

### Huile de protection de la peau

On mélange les ingrédients suivants en chauffant éventuellement à 40-45°C pour homogénéiser :

| | |
|---|---|
| - Bis (3,5-diisopropylsalicylate) de Cu II | 1 g |
| - Parfum qs | |
| - Huile de colza qsp | 100 g |

### EXEMPLE 2

### Crème de protection de la peau

| | |
|---|---|
| - Bis (3,5-diisopropylsalicylate) de Cu II | 0,5 g |
| - Mélange d'alcool cétylstéarylique (80%) et d'alcool cétylstéarylique oxyéthyléné à 33 moles d'oxyde d'éthylène (20%) | 7 g |
| - Mono et distéarate de glycérol non auto-émulsionnable | 2 g |
| - Alcool cétylique | 1,5 g |
| - Huile de vaseline | 15 g |
| - Glycérol | 10 g |
| - Sorbitol | 5 g |
| - Parfum, conservateur qs | |
| - Eau qsp | 100 g |

Cette crème est préparée selon les techniques classiques de préparation d'émulsions en dissolvant le complexe cuivrique dans les corps gras et les émulsionnants, en chauffant cette phase grasse vers 75-80°C et en ajoutant, sous vive agitation, l'eau, le glycérol et le sorbitol chauffés également à 75-80°C. On maintient l'agitation pendant 10 à 15 minutes, puis on laisse refroidir sous agitation modérée et vers 40°C, on ajoute parfum et conservateur.

### EXEMPLE 3

### Crème de protection de la peau

| | |
|---|---|
| - Alcool cétylstéarylique oxyéthyléné à 15 moles d'oxyde d'éthylène | 3 g |
| - Mélange de mono et distéarate de glycérol non auto-émulsionnable | 4,8 g |
| - Alcool myristique | 4,5 g |
| - Huile de vaseline | 18 g |
| - Bis (3,5-diisopropylsalicylate) de Cu II | 0,5 g |
| - p-(diméthylamino) benzoate de 2-éthylhexyle "ESCALOL 507" de VAN DYK | 2,75g |
| - 3-benzylidène-DL-camphre | 2 g |
| - Propylène glycol | 6 g |
| - Conservateur, parfum qs | |
| - Eau qsp | 100 g |

Cette crème est préparée de la même façon que dans l'exemple 2.

### EXEMPLE 4

### Huile de protection de la peau

| | |
|---|---|
| - Bis (3,5-diisopropylsalicylate) de Cu II | 0,2 g |
| - P-méthoxycinnamate d'octyle vendu par la Société GIVAUDAN sous la dénomination "PARSOL MCX" | 5 g |
| - Huile de vaseline | 40 g |
| - Conservateur, parfum qs | |
| - Benzoate d'alcools primaires en C₁₂-C₁₅ vendu sous l'appellation "FINSOLV TN" par la Société FINETEX qsp | 100 g |

Cette huile est préparée de la même façon que dans l'exemple 1.

### Description pour l'Etat contractant suivant : ES

La présente invention est relative à une composition cosmétique contenant un complexe cuivrique de l'acide 3,5-diisopropyl salicylique.

Il est bien connu que la peau, soumise à l'irradiation selaire, subit différentes modifications et en particulier certains dommages.

Parmi les dommages causés à la peau par le soleil, le plus spectaculaire est incontestablement le coup de soleil ou érythème solaire qui apparait, selon l'intensité et la durée d'irradiation, entre la deuxième et la vingt quatrième heure après l'exposition au soleil. L'érythème solaire peut se manifester par un léger rosissement de la peau, mais peut aussi conduire à de véritables brûlures accompagnées de troubles importants de l'état général.

Le rayonnement solaire peut également provoquer des modifications cellulaires au niveau de la peau. On observe notamment la formation dans l'épiderme de kératinocytes dégradés, voire détruits par les radiations UV, qui sont des cellules dégénérées et nécrosées sous l'action des radiations solaires, généralement connues sous l'appellation de "sun burn cells"

Le domaine du rayonnement solaire responsable de ces phénomènes se situe dans les radiations ultraviolettes, c'est-à-dire dans la gamme de longueurs d'onde comprises entre 280 et 400 nm. On a donc cherché à protéger la peau par application de substances présentant un maximum d'absorption dans cette zone. Ces substances constituent ce que l'on appelle des filtres solaires. Selon la quantité de filtre solaire appliquée sur la peau, une partie plus ou moins importante des rayons nocifs est arrêtée, ce qui diminue d'autant l'apparition des dommages causés à la peau.

De nombreux filtres solaires ont déjà été préconisés, de natures chimiques diverses et présentant des caractéristiques physicochimiques variées, en particulier en ce qui concerne la solubilté (produits hydrosolubles ou liposolubles). Ces composés assurent ce que l'on peut appeler une protection primaire.

Cependant, l'absorption du rayonnement ultraviolet par les filtres n'est jamais totale. Il subsiste donc une quantité résiduelle de radiations énergétiques qui atteint la peau. Ainsi, les filtres solaires atténuent les dommages créés au niveau de la peau sans les supprimer totalement.

Il est donc souhaitable d'associer aux filtres solaires ou de les remplacer par des produits susceptibles de diminuer les dommages causés à la peau par un phénomène autre qu'une simple filtration.

La demanderesse a découvert que l'application sur la peau d'un complexe formé de deux molécules d'acide 3,5-diisopropyl salicylique et d'un atome de cuivre à savoir le bis(3,5-diisopropylsalicylate) de cuivre II, de formule 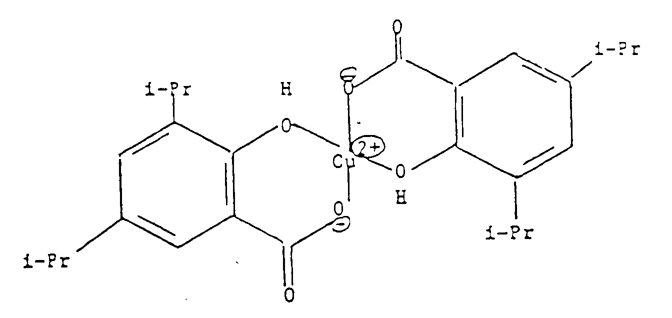 permettait de façon surprenante de réduire l'intensité de l'érythème solaire et le nombre de kérationocytes dégradés, sans que ces propriétés soient dues à un net pouvoir absorbant dans le domaine du rayonnement ultraviolet. Ces propriétés persistent en présence de filtres solaires.

Le complexe cuivrique de l'acide 3,5-diisopropyl salicylique est un composé connu dont la synthèse est décrite dans "Journal of Medicinal Chemistry", 1976, Vol. 19, n° 1, p 135-148. Ce document ainsi que le brevet US 4 221 785 décrivent également les propriétés anti-inflammatoires et anti-ulcère de ce complexe cuivrique pouvant être administré par voie parentérale, en particulier sous-cutanée, et par voie orale. La demande de brevet international WO 84/04922 décrit ce composé comme agent anti-tumoral administré par voie intramusculaire.

Cependant, l'application de ce complexe cuivrique par voie topique dans le domaine cosmétique, qui fait l'objet de la présente invention, n'est préconisée dans aucun des documents de l'art antérieur cités ci-dessus, Or, la demanderesse a pu démontrer que l'action de ce complexe cuivrique vis-à-vis des radiations UV s'exerce essentiellement à la surface de la peau.

La présente invention a pour objet une composition cosmétique utile pour la protection de la peau centre le rayonnement ultraviolet de longueurs d'onde comprises entre 280 et 400 nm comprenant, dans un support cosmétiquement acceptable contenant au moins une phase grasse, une quantité efficace de bis(3,5-diisopropylsalicylate) de cuivre II.

Un autre objet de l'invention est constitué par une composition cosmétique de protection de la peau comprenant, dans un support cosmétiquement acceptable contenant au moins une phase grasse, ledit complexe cuivrique de l'acide 3,5-diisopropyl salicylique et au moins un filtre solaire absorbant les rayons ultraviolets de longueurs d'onde comprises entre 280 et 400 nm.

D'autres objets et caractéristiques de l'invention apparaîtront à la lecture de la description qui va suivre.

La composition cosmétique selon l'invention contient, dans une phase grasse, le complexe cuivrique de l'acide 3,5-diisopropyl salicylique dans une gamme de concentrations allant de 0,01 à 5% et de préférence de 0,05 à 1% en poids, sur la base du poids total de la composition. Il est à noter que, bien que coloré en solution, le complexe cuivrique ci-dessus ne colore pas la peau aux concentrations utilisées.

En raison du caractère liposoluble du complexe cuivrique utilisé, les compositions cosmétiques selon l'invention contiennent au moins une phase grasse. Elles peuvent se présenter sous forme d'huiles, de lotions oléoalcooliques, d'émulsions telles que des crèmes ou des laits, de gels gras ou olécalcooliques, de bâtonnets solides ou être conditionnées en aérosol.

La phase grasse est constituée de corps gras tels que les huiles ou les cires minérales, animales ou végétales, les acides gras, les triglycérides d'acides gras ayant de 6 à 18 atomes de carbone et les alcools gras.

Outre la phase grasse, le support cosmétique peut aussi contenir des monoalcools ou polyalcools inférieurs contenant de 1 à 6 atomes de carbone ou bien de l'eau et des mélanges de ces différents constituants.

Les mono- ou polyalcools plus particulièrement préférés sont choisis parmi l'éthanol, l'isopropanol, le propylène glycol, le glycérol et le sorbitol. Les mélanges hydroalcooliques sont de préférence des mélanges d'eau et d'éthanol,

A titre de corps gras, on peut utiliser :
- des huiles minérales telles que l'huile de vaseline ;
- des huiles animales telles que les huiles de baleine, de phoque, de menhaden, de foie de flétan, de morue, de thon, de tortue, de suif, de pied de boeuf, de pied de cheval, de pied de mouton, de vison, de loutre, de marmotte ;
- des huiles végétales telles que des huiles d'amande, d'arachide, de germes de blé, d'olive, de germes de maïs, de jojoba, de sésame, de tournesol, de palme, de noix, de colza.

On peut également utiliser comme corps gras : la vaseline, la paraffine, la lanoline hydrogénée, la lanoline acétylée et les huiles de silicone.

On peut utiliser comme cires la cire de sipol, la cire de lanoline, la cire d'abeille, la cire de candellila, la cire microcristalline, la cire de carnauba, le spermaceti, le beurre de cacao, le beurre de karité, les cires de silicones, les huiles hydrogénées concrètes à 25°C, les sucroglycérides, les oléates, myristates, linoléates et stéarates de Ca, Mg, Zn et Al.

Parmi les alcools gras, on peut citer les alcools laurique, cétylique, myristique, stéarique, palmitique et oléique.

La composition cosmétique selon l'invention peut également renfermer des émulsifiants non-ioniques, anioniques, cationiques ou amphotères.

Il peut aussi être avantageux d'utiliser des épaississants tels que les dérivés de cellulose, les dérivés d'acide polyacrylique, les gommes de guar ou de caroube, les gommes de Xanthane.

La composition cosmétique selon l'invention peut également contenir des adjuvants habituellement utilisés en cosmétique et notamment des produits hydratants, des adoucissants, des conservateurs, des colorants, des opacifiants, des parfums et des propulseurs ; les propulseurs sont des propulseurs classiques tels que les alcanes, les fluoroalcanes et les chlorofluoroalcanes.

La composition cosmétique selon l'invention possède un pH compris entre 4 et 9, et de préférence entre 5,5 et 8, qui peut éventuellement être ajusté à l'aide d'un agent de régulation du pH.

La composition cosmétique selon l'invention peut également contenir, en association avec le complexe cuivrique de l'acide 3,5-diisopropyl salicylique, au moins un filtre solaire absorbant les radiations de longueurs d'onde comprises entre 280 et 400 nm.

La concentration totale en filtre(s) solaire(s) est comprise entre 0,5 et 20% du poids total de la composition.

Ces filtres solaires sont choisis parmi les dérivés :
- de l'acide p-aminobenzoïque ;
- de l'acide cinnamique ;
- du benzylidène camphre ;
- de l'acide salicylique ;
- de l'acide anthranilique ;
- de l'acide urocanique ;
- de la benzophénone ;
- du dibenzoylméthane ;
- du benzotriazole ;
- de l'acide benzimidazole sulfonique.

Ils sont en particulier choisis parmi ceux cités dans l'article : "UV absorbers in sun cosmetics 1978", D.H. LIEM and L.T.H. HILDERINK, International Journal of Cosmetic Science 1, 341-361 (1979).

On peut également utiliser des polymères filtres comme ceux décrits dans les brevets français 2 237 912 et 2 359 857 de la demanderesse.

Une forme de réalisation de l'invention est une émulsion sous forme de crème ou de lait comprenant, en plus du complexe curvrique, des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation de la composition cosmétique de l'invention est constituée par des huiles à base d'huiles et cires naturelles ou synthétiques, de lanoline et d'esters d'acides gras, notamment de triglycérides d'acides gras, ou par des lotions oléoalcooliques à base d'huiles, de cires, d'esters d'acides gras tels que les triglycérides d'acides gras et d'alcools inférieurs tels que l'éthanol ou de glycols tels que le propylène glycol ou de polyols comme le glycérol.

La composition cosmétique de l'invention peut également être un gel gras comprenant des huiles ou des cires et un épaississant tel que la silice ; les gels oléoalcooliques comprennent en outre un ou plusieurs alcools ou polyols inférieurs tels que I'éthanol, le propylène glycol ou le glycérol.

Les bâtonnets solides sont constitués de cires et d'huiles naturelles ou synthétiques, d'alcools gras, d'esters d'acides gras, de lanoline et autres corps gras.

Le procédé de préparation d'une composition cosmétique selon l'invention consiste, dans une première étape, à dissoudre 0,01 à 5% en poids, sur la base du poids total de la composition, de bis(3,5-diisopropylsalicylate) de cuivre II dans une phase grasse préalablement chauffée à 40-90°C comprenant au moins un constituant choisi parma les huiles ou cires minérales, animales et végétales, les acides gras, les triglycérides d'acides gras ayant de 6 à 18 atomes de carbone et les alcools gras et dans une deuxième étape, à ajouter au moins un adjuvant cosmétique choisi parmi les monoalcools ou polyalcools inférieurs contenant 1 à 6 atomes de carbone, l'eau ou les mélanges de ces composés, les émulsifiants non-ioniques, anioniques, cationiques ou amphotères, les épaississants, les produits hydratants, les adoucissants, les conservateurs, les colorants, les opacifiants, les agents de régulation du pH, les propulseurs et les parfums.

Pour la préparation d'une composition cosmétique en émulsion sous forme de crème ou de lait protecteur, on ajoute, sous vive agitation, à la phase grasse préalablement chauffée à 75-80°C dans laquelle on a dissous le bis(3,5-diisopropylsalicylate) de cuivre II et les émulsifiants, la phase aqueuse chauffée à la même température, on maintient l'agitation pendant 10 à 15 minutes, puis on laisse refroidir sous agitation modérée et vers 40°C, on ajoute parfum et conservateur.

Pour la préparation d'une composition cosmétique sous forme d'un gel gras, d'une huile ou d'une lotion ou d'un gel oléoalcoolique, on chauffe à 40-50°C le mélange de bis(3,5-diisopropylsalicylate) de cuivre II, de la phase grasse ou oléoalcoolique et des adjuvants cosmétiques.

Les exemples suivants sont destinés à illustrer l'invention sans pour autant présenter un caractère limitatif.

### EXEMPLE 1

### Huile de protection de la peau

On mélange les ingrédients suivants en chauffant éventuellement à 40-45°C pour homogénéiser :

| | |
|---|---|
| - Bis (3,5-diisopropylsalicylate) de Cu II | 1 g |
| - Parfum qs | |
| - Huile de colza qsp | 100 g |

### EXEMPLE 2

### Crème de protection de la peau

| | |
|---|---|
| - Bis (3,5-diisopropylsalicylate) de Cu II | 0,5 g |
| - Mélange d'alcool cétylstéarylique (80%) et d'alcool cétylstéarylique oxyéthyléné à 33 moles d'oxyde d'éthylène (20%) | 7 g |
| - Mono et distéarate de glycérol non auto-émulsionnable | 2 g |
| - Alcool cétylique | 1,5 g |
| - Huile de vaseline | 15 g |
| - Glycérol | 10 g |
| - Sorbitol | 5 g |
| - Parfum, conservateur qs | |
| - Eau qsp | 100 g |

Cette crème est préparée selon les techniques classiques de préparation d'émulsions en dissolvant le complexe cuivrique dans les corps gras et les émulsionnants, en chauffant cette phase grasse vers 75-80°C et en ajoutant, sous vive agitation, l'eau, le glycérol et le sorbitol chauffés également à 75-80°C. On maintient l'agitation pendant 10 à 15 minutes, puis on laisse refroidir sous agitation modérée et vers 40°C, on ajoute parfum et conservateur.

### EXEMPLE 3

### Crème de protection de la peau

| | |
|---|---|
| - Alcool cétylstéarylique oxyéthyléné à 15 moles d'oxyde d'éthylène | 3 g |
| - Mélange de mono et distéarate de glycérol non auto-émulsionnable | 4,8 g |
| - Alcool myristique | 4,5 g |
| - Huile de vaseline | 18 g |
| - Bis (3,5-diisopropylsalicylate) de Cu II | 0,5 g |
| - p-(diméthylamino) benzoate de 2-éthylhexyle "ESCALOL 507" de VAN DYK | 2,75g |
| - 3-benzylidène-DL-camphre | 2 g |
| - Propylène glycol | 6 g |
| - Conservateur, parfum qs | |
| - Eau qsp | 100 g |

Cette crème est préparée de la même façon que dans l'exemple 2.

### EXEMPLE 4

### Huile de protection de la peau

| | |
|---|---|
| - Bis (3,5-diisopropylsalicylate) de Cu II | 0,2 g |
| - P-méthoxycinnamate d'octyle vendu par la Société GIVAUDAN sous la dénomination "PARSOL MCX" | 5 g |
| - Huile de vaseline | 40 g |
| - Conservateur, parfum qs | |
| - Benzoate d'alcools primaires en C₁₂-C₁₅ vendu sous l'appellation "FINSOLV TN" par la Société FINETEX qsp | 100 g |

Cette huile est préparée de la même façon que dans l'exemple 1.

## Revendications (Revendications pour l'(les) Etat(s) contractant(s) suivant(s): BE, CH, DE, FR, GB, IT, LI, NL, SE, AT, GR)

1. Composition cosmétique pour la protection de la peau contre le rayonnement ultraviolet de longueurs d'onde comprises entre 280 et 400 nm, caractérisée par le fait qu'elle comprend, dans un support cosmétique contenant au moins une phase grasse, une quantité efficace de bis(3,5-diisopropyl-salicylate) de cuivre II de formule :

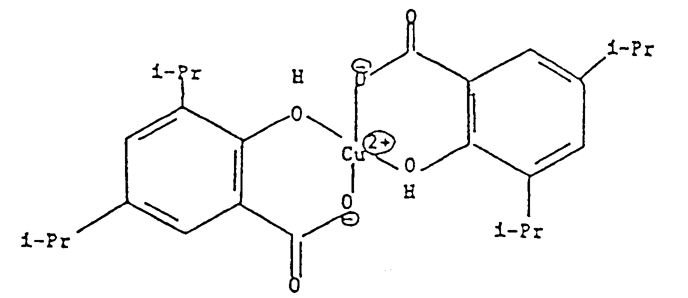

2. Composition cosmétique selon la revendication 1, caractérisée par le fait qu'elle contient 0,01 à 5% en poids, et de préférence 0,05 à 1% en poids, de bis(3,5-diisopropylsalicylate) de cuivre II.

3. Composition cosmétique selon la revendication 1 ou 2, caractérisée par le fait qu'elle contient en outre un ou plusieurs filtres solaires absorbant les rayons ultraviolets dans la gamme de longueurs d'onde allant de 280 à 400 nm.

4. Composition cosmétique selon la revendication 3, caractérisée par le fait que les filtres solaires sont présents dans des proportions comprises entre 0,5 et 20% en poids par rapport au poids total de la composition.

5. Composition cosmétique selon la revendication 3 ou 4, caractérisée par le fait que les filtres solaires sont choisis parmi les dérivés de l'acide p-aminobenzoïque, de l'acide cinnamique, du benzylidène camphre, de l'acide salicylique, de l'acide anthranilique, de l'acide urocanique, de la benzophénone, du dibenzoylméthane, du benzotriazole et de l'acide benzimidazole sulfonique.

6. Composition cosmétique selon l'une quelconque des revendications 1 à 5, caractérisée par le fait qu'elle se présente sous forme d'huile, de lotion oléoalcoolique, d'émulsion telle qu'une crème ou un lait, de gel gras ou oléoalcoolique, de bâtonnet solide ou d'aérosol.

7. Composition cosmétique selon l'une quelconque des revendications 1 à 6, caractérisée par le fait que la phase grasse comprend des huiles ou cires minérales, animales et végétales, des acides gras, des triglycérides d'acides gras ayant de 6 à 18 atomes de carbone et des alcools gras.

8. Composition cosmétique selon l'une quelconque des revendications 1 à 7, caractérisée par le fait que le support cosmétique contient des monoalcools ou polyalcools inférieurs contenant de 1 à 6 atomes de carbone, de l'eau ou des mélanges de ces composés.

9. Composition cosmétique selon l'une quelconque des revendications 1 à 8, caractérisée par le fait qu'elle contient en outre des adjuvants cosmétiques choisis parmi les émulsifiants non-ioniques, anioniques, cationiques ou amphotères, les épaississants, les produits hydratants, les adoucissants, les conservateurs, les colorants, les opacifiants, les agents de régulation du pH, les propulseurs et les parfums.

## Revendications (Revendications pour l'(les) Etat(s) contractant(s) suivant(s): ES)

1. Composition cosmétique pour la protection de la peau contre le rayonnement ultraviolet de longueurs d'onde comprises entre 280 et 400 nm, caractérisée par le fait quelle comprend, dans un support cosmétique contenant au moins une phase grasse, une quantité efficace de bis(3,5-diisopropyl-salicylate) de cuivre II de formule :

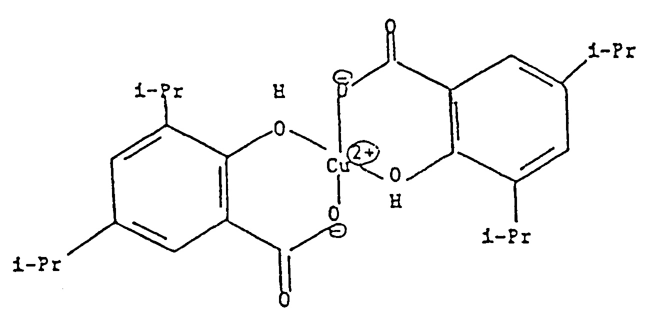

2. Composition cosmétique selon la revendication 1, caractérisée par le fait quelle contient 0,01 à 5% en poids, et de préférence 0,05 à 1% en poids, de bis(3,5-diisopropylsalicylate) de cuivre II.

3. Composition cosmétique selon la revendication 1 ou 2, caractérisée par le fait qu'elle contient en outre un ou plusieurs filtres solaires absorbant les rayons ultraviolets dans la gamme de longueurs d'onde allant de 280 à 400 nm.

4. Composition cosmétique selon la revendication 3, caractérisée par le fait que les filtres solaires sont présents dans des proportions comprises entre 0,5 et 20% en poids par rapport au poids total de la composition.

5. Composition cosmétique selon la revendication 3 ou 4, caractérisée par le fait que les filtres solaires sont choisis parmi les dérivés de l'acide p-aminobenzoïque, de l'acide cinnamique, du benzylidène camphre, de l'acide salicylique, de l'acide anthranilique, de l'acide urocanique, de la benzophénone, du dibenzoylméthane, du benzotriazole et de l'acide benzimidazole sulfonique.

6. Composition cosmétique selon l'une quelconque des revendications 1 à 5, caractérisée par le fait qu'elle se présente sous forme d'huile, de lotion oléoalcoolique, d'émulsion telle qu'une crème ou un lait, de gel gras ou oléoalcoolique, de bâtonnet solide ou d'aérosol.

7. Composition cosmétique selon l'une quelconque des revendications 1 à 6, caractérisée par le fait que la phase grasse comprend des huiles ou cires minérales, animales et végétales, des acides gras, des triglycérides d'acides gras ayant de 6 à 18 atomes de carbone et des alcools gras.

8. Composition cosmétique selon l'une quelconque des revendications 1 à 7, caractérisée par le fait que le support cosmétique contient des monoalcools ou polyalcools inférieurs contenant de 1 à 6 atomes de carbone, de l'eau ou des mélanges de ces composés.

9. Composition cosmétique selon l'une quelconque des revendications 1 à 8, caractérisée par le fait qu'elle contient en outre des adjuvants cosmétiques choisis parmi les émulsifiants non-ioniques, anioniques, cationiques ou amphotères, les épaississants, les produits hydratants, les adoucissants, les conservateurs, les colorants, les opacifiants, les agents de régulation du pH, les propulseurs et les parfums.

10. Procédé de préparation d'une composition cosmétique pour la protection de la peau contre le rayonnement ultraviolet de longueurs d'ondes comprises entre 280 et 400 nm, caractérisé par le fait que dans une première étape, on dissout 0,01 à 5% en poids, sur la base du poids total de la composition, de bis(3,5-diisopropylsalicylate) de cuivre II dans une phase grasse préalablement chauffée à 40-90°C comprenant au moins un constituant choisi parmi les huiles ou cires minérales, animales et végétales, les acides gras, les triglycérides d'acides gras ayant de 6 à 18 atomes de carbone et les alcools gras et dans une deuxième étape, on ajoute au moins un adjuvant cosmétique choisi parmi les monoalcools ou polyalcools inférieurs contenant 1 à 6 atomes de carbone, l'eau ou les mélanges de ces composés, les émulsifiants non-ioniques, anioniques, cationiques ou amphotères, les épaississants, les produits hydratants, les adoucissants, les conservateurs, les colorants, les opacifiants, les agents de régulation du pH, les propulseurs et les parfums.

11. Procédé selon la revendication 10, caractérisé par le fait qu'on dissout dans la phase grasse 0,05 à 1% en poids, sur la base du poids total de la composition, de bis(3,5-diisopropylsalicylate) de cuivre II.

12. Procédé selon la revendication 11 ou 12, caractérisé par le fait qu'on ajoute à la composition cosmétique 0,5 à 20% en poids sur la base du poids total de la composition, d'un ou plusieurs filtres solaires absorbant les rayons ultraviolets dans la gamme de longueurs d'ondes allant de 280 à 400 nm, choisis parmi les dérivés de l'acide p-aminobenzoïque, de l'acide cinnamique, du benzylidène camphre, de l'acide salicylique, de l'acide anthranilique, de l'acide urocanique, de la benzophénone, du dibenzoylméthane, du benzotriazole et de l'acide benzimidazole sulfonique.

13. Procédé selon l'une quelconque des revendications 10 à 12 de préparation d'une composition cosmétique en émulsion sous forme de crème ou de lait protecteur, caractérisé par le fait qu'on ajoute, sous vive agitation, à la phase grasse préalablement chauffée à 75-80°C dans laquelle on a dissous le bis(3,5-diisopropylsalicylate) de cuivre II et les émulsifiants, la phase aqueuse chauffée à la même température, on maintient l'agitation pendant 10 à 15 minutes, puis on laisse refroidir sous agitation modérée et vers 40°C, on ajoute parfum et conservateur.

14. Procédé selon l'une quelconque des revendications 10 à 12 de préparation d'une composition cosmétique sous forme d'un gel gras, d'une huile ou d'une lotion ou d'un gel oléoalcoolique, caractérisé par le fait qu'on chauffe à 40-50°C le mélange de bis(3,5-diisopropylsalicylate) de cuivre II, de la phase grasse ou oléoalcoolique et des adjuvants cosmétiques.

## Claims (Claims for the following Contracting State(s): BE, CH, DE, FR, GB, IT, LI, NL, SE, AT, GR)

1. Cosmetic composition for protecting the skin against ultraviolet radiation of wavelengths between 280 and 400 nm, characterized in that it comprises, in a cosmetic carrier containing at least one fatty phase, an effective quantity of copper(II) bis-(3,5-diisopropylsalicylate) of the formula

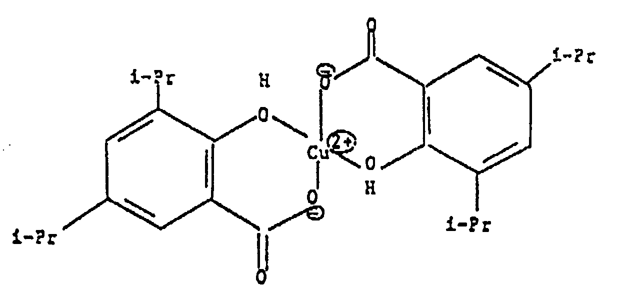

2. Cosmetic composition according to Claim 1, characterized in that it contains 0.01 to 5 % by weight, and preferably 0.05 to 1 % by weight, of copper(II) bis-(3,5-diisopropylsalicylate).

3. Cosmetic composition according to Claim 1 or 2, characterized in that it furthermore contains one or more solar filters which absorb ultraviolet rays in the range of wavelengths of from 280 to 400 nm.

4. Cosmetic composition according to Claim 3, characterized in that the solar filters are present in proportions of between 0.5 and 20 % by weight relative to the total weight of the composition.

5. Cosmetic composition according to Claim 3 or 4, characterized in that the solar filters are chosen from among the derivatives of p-aminobenzoic acid, of cinnamic acid, of benzylidene-camphor, of salicylic acid, of anthranilic acid, of urocanic acid, of benzophenone, of dibenzoylmethane, of benzotriazole and of benzimidazolesulphonic acid.

6. Cosmetic composition according to any one of Claims 1 to 5, characterized in that it is in the form of an oil, an oleo-alcoholic lotion, an emulsion such as a cream or a milk, a fatty or oleo-alcoholic gel, a solid stick or an aerosol.

7. Cosmetic composition according to any one of Claims 1 to 6, characterized in that the fatty phase comprises inorganic, animal and vegetable oils or waxes, fatty acids, triglycerides of fatty acids having from 6 to 18 carbon atoms, and fatty alcohols.

8. Cosmetic composition according to any one of Claims 1 to 7, characterized in that the cosmetic carrier contains lower monoalcohols or polyalcohols, containing from 1 to 6 carbon atoms, water or mixtures of those compounds.

9. Cosmetic composition according to any one of Claims 1 to 8, characterized in that it furthermore contains cosmetic adjuvants chosen from among non-ionic, anionic, cationic or amphoteric emulsifiers, thickeners, moisturizing products, softeners, preservatives, colorants, opacifiers, pH-regulating agents, propellants and perfumes.

## Claims (Claims for the following Contracting State(s): ES)

1. Cosmetic composition for protecting the skin against ultraviolet radiation of wavelengths between 280 and 400 nm, characterized in that it comprises, in a cosmetic carrier containing at least one fatty phase, an effective quantity of copper(II) bis-(3,5-diisopropylsalicylate) of the formula

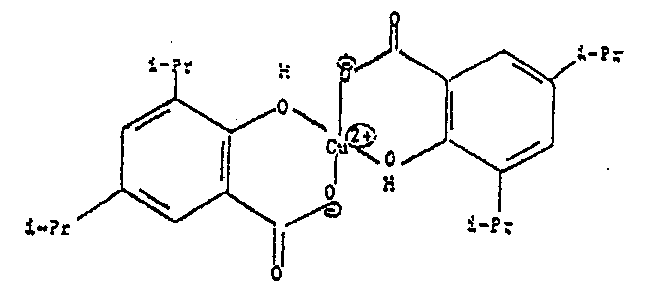

2. Cosmetic composition according to Claim 1, characterized in that it contains 0.01 to 5 % by weight, and preferably 0.05 to 1 % by weight, of copper(II) bis-(3,5-diisopropylsalicylate).

3. Cosmetic composition according to Claim 1 or 2, characterized in that it furthermore contains one or more solar filters which absorb ultraviolet rays in the range of wavelengths of from 280 to 400 nm.

4. Cosmetic composition according to Claim 3, characterized in that the solar filters are present in proportions of between 0.5 and 20 % by weight relative to the total weight of the composition.

5. Cosmetic composition according to Claim 3 or 4, characterized in that the solar filters are chosen from among the derivatives of p-aminobenzoic acid, of cinnamic acid, of benzylidene-camphor, of salicylic acid, of anthranilic acid, of urocanic acid, of benzophenone, of dibenzoylmethane, of benzotriazole and of benzimidazolesulphonic acid.

6. Cosmetic composition according to any one of Claims 1 to 5, characterized in that it is in the form of an oil, an oleo-alcoholic lotion, an emulsion such as a cream or a milk, a fatty or oleo-alcoholic gel, a solid stick or an aerosol.

7. Cosmetic composition according to any one of Claims 1 to 6, characterized in that the fatty phase comprises inorganic, animal and vegetable oils or waxes, fatty acids, triglycerides of fatty acids having from 6 to 18 carbon atoms, and fatty alcohols.

8. Cosmetic composition according to any one of Claims 1 to 7, characterized in that the cosmetic carrier contain, lower monoalcohols or polyalcohols, containing from 1 to 6 carbon atoms, water or mixtures of these compounds.

9. Cosmetic composition according to any one of Claims 1 to 8, characterized in that it furthermore contains cosmetic adjuvants chosen from among non-ionic, anionic, cationic or amphoteric emulsifiers, thickeners, moisturizing products, softeners, preservatives, colorants, opacifiers, pH-regulating agents, propellants and perfumes.

10. Process for the preparation of a cosmetic composition for the protection of the skin from ultraviolet radiation of wavelengths of between 280 and 400 nm, characterized in that in a first stage 0.01 to 5 % by weight, based on the total weight of the composition, of copper(II) bis-(3,5-diisopropylsalicylate) are dissolved in a fatty phase, heated beforehand to 40-90°C, comprising at least one constituent chosen from among mineral, animal and vegetable oils or waxes, fatty acids, triglycarides of fatty acids having from 6 to 18 carbon atoms and fatty alcohols, and, in a second stage, there is added at least one cosmetic adjuvant chosen from among lower monoalcohols or polyalcohols containing 1 to 6 carbon atoms, water or mixtures of these compounds, nonionlc, anionic, cationic or amphoteric emulsifiers, thickeners, moisturizing products, softeners, preservatives, colorants, opacifiers, pH regulators, propellants and perfumes.

11. Process according to Claim 10, characterized in that 0.05 to 1 % by weight, based on the total weight of the composition, of copper(II) bis-(3,5-diisopropylsalicylate) is dissolved in the fatty phase.

12. Process according to Claim 10 or 11, characterised in that there are added to the cosmetic composition 0.5 to 20 % by weight, based on the total weight of the composition, of one or more solar filters which absorb ultraviolet rays in the range of wavelengths of from 280 to 400 nm, chosen from among the derivatives of p-aminobenzoic acid, of cinnamic acid, of benzylidene-camphor, of salicylic acid, of anthranilic acid, of urocanic acid, of benzophenone, of dibenzoylmethane, of benzotriazole and of benzimidazole-sulphonic acid.

13. Process according to any one of Claims 10 to 12, for the preparation of a cosmetic composition in emulsion, in the form of a protective milk or cream, characterized in that there is added with vigorous stirring to the fatty phase, which has been heated beforehand to 75-80°C and in which the copper(II) bis-(3,5-diisopropylsalicylate) and the emulsifiers have been dissolved, the aqueous phase heated to the same temperature, stirring is continued for 10 to 15 minutes, and thereafter the mixture in allowed to cool with moderate stirring and at about 40°C perfume and preservative are added.

14. Process according to any one of Claims 10 to 12 for the preparation of a cosmetic composition in the form of a fatty gel, an oil, a lotion or an oily-alcoholic gel, characterized in that the mixture of copper(II) bis-(3,5-diisopropylsalicylate), the fatty or oily-alcoholic phase and the cosmetic adjuvants is heated to 40-50°C.

## Patentansprüche (Patentansprüche für folgende(n) Vertragsstaat(en): BE, CH, DE, FR, GB, IT, LI, NL, SE, AT, GR)

1. Kosmetische Zusammensetzung zum Schutz der Haut gegen ultraviolette Strahlung der Wellenlänge zwischen 280 und 400 nm, dadurch gekennzeichnet, daß sie in einem kosmetischen Träger, der mindestens eine Fettphase enthält, eine wirksame Menge von Kupfer (II)- bis (3,5-Diisopropylsalicylat) der Formel 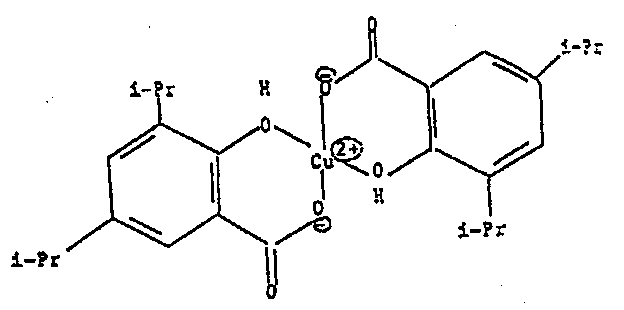 enthält.

2. Kosmetische Zusammensetzung nach Anspruch 1, dadurch gekennzeichnet, daß sie 0.01 bis 5 Gew. %, und vorzugsweise 0.05 bis 1 Gew. % von Kupfer (II)- bis (3,5-Diisopropylsalicylat) enthält.

3. Kosmetische Zusammensetzung nach Anspruch 1 oder 2, dadurch gekennzeichnet, daß sie einen oder, mehrere Sonnenfilter enthält, die ultraviolette Strahlen im Wellenlängenbereich von 280 bis 400 nm absorbieren.

4. Kosmetische Zusammensetzung nach Anspruch 3, dadurch gekennzeichnet, daß die Sonnenfilter in Anteilen zwischen 0.5 und 20 Gew. %, bezogen auf das Gesamtgewicht der Zusammensetzung, vorhanden sind.

5. Kosmetische Zusammensetzung nach Anspruch 3 oder 4, dadurch gekennzeichnet, daß die Sonnenfilter ausgewählt sind aus den Derivaten der p-Aminobenzosäure, Zimtsäure, Benzylidenkampfer, Salicylsäure, Anthranilsäure, Urocansäure, von Benzophenon, von Dibenzoylmethan, von Benzotriazol und von Benzimidazolsulfonsäure.

6. Kosmetische Zusammensetzung nach einem der Ansprüche 1 bis 5, dadurch gekennzeichnet, daß sie in Form einen Öls, einer ölalkoholischen Lotion, einer Emulsion wie z.B. einer Creme oder einer Milch, eines Fettgels oder ölalkoholischen Gels, eines festen Stiftes oder als Aerosol vorliegt.

7. Kosmetische Zusammensetzung nach einem der Ansprüche 1 bis 6, dadurch gekennzeichnet, daß die Fettphase mineralische, tierische und pflanzliche Öle oder Wachse, Fettsäuren, Fettsäuretriglyceride mit 6 bis 18 Kohlenstoffatomen und Fettalkohole umfaßt.

8. Kosmetische Zusammensetzung nach einem der Ansprüche 1 bis 7, dadurch gekennzeichnet, daß der kosmetische Träger niedrige Monoalkohole oder Polyalkohole mit 1 bis 6 Kohlenstoffatomen, Wasser oder eine Mischung dieser Verbindungen enthält.

9. Kosmetische Zusammensetzung nach einem der Ansprüche 1 bis 8, dadurch gekennzeichnet, daß sie außerdem kosmetische Hilfsstoffe enthält, ausgewählt aus nichtionischen, anionischen, kationischen oder amphoteren Emulgatoren, Verdickungsmitteln, Hydratationsmitteln, Weichmachern, Konservierungsmitteln, Farbstoffen, Trübungsmitteln, Mitteln zur Regulierung des pH-Werts, Treibmitteln und Parfüms.

## Patentansprüche (Patentansprüche für folgende(n) Vertragsstaat(en): ES)

1. Kosmetische Zusammensetzung zum Schutz der Haut gegen ultraviolette Strahlung der Wellenlänge zwischen 280 und 400 nm, dadurch gekennzeichnet, daß sie in einem kosmetischen Träger, der mindestens eine Fettphase enthält, eine wirksame Menge von Kupfer (II)- bis (3,5-Diisopropyl-salicylat) der Formel 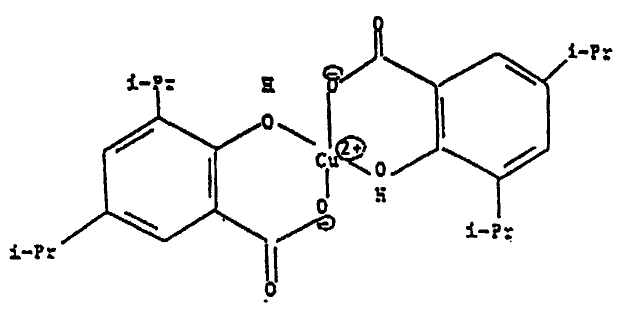 enthält.

2. Kosmetische Zusammentsetzung nach Anspruch 1, dadurch gekennzeichnet, daß sie 0.01 bis 5 Gew. %, und vorzugsweise 0.05 bis 1 Gew. % Von Kupfer (II)- bis (3,5-Diisopropylsalicylat) enthält.

3. Kosmetische Zusammensetzung nach Anspruch 1 oder 2, dadurch gekennzeichnet, daß sie zusätzlich einen oder mehrere Sonnenfilter enthält, die ultraviolette Strahlen im Wellenlängenbereich von 280 bis 400 nm absorbieren.

4. Kosmetische Zusammensetzung nach Anspruch 3, dadurch gekennzeichnet, daß die Sonnenfilter in Anteilen zwischen 0.5 und 20 Gew. %, bezogen auf das Gesamtgewicht der Zusammensetzung, vorhanden sind.

5. Kosmetische Zusammensetzung nach Anspruch 3 oder 4, dadurch gekennzeichnet, daß die Sonnenfilter ausgewählt sind aus den Derivaten der p-Aminobenzoesäure, Zimtsäure, Benzylidenkampfer, Salicylsäure, Anthranilsäure, Urocansäure, von Benzophenon, von Dibenzoylmethan, von Benzotriazol und von Benzimidazolsulfonsäure.

6. Kosmetische Zusammensetzung nach einem der Ansprüche 1 bis 5, dadurch gekennzeichnet, daß sie in Form eines Öls, einer ölalkoholischen Lotion, einer Emuision wie z.B. einer Creme oder einer Milch, eines Fettgels oder ölalkoholischen Gels, eines festen Stiftes oder als Aerosol vorliegt.

7. Kosmetische Zusammensetzung nach einem der Ansprüche 1 bis 6, dadurch gekennzeichnet, daß die Fettphase mineralische, tierische und pflanzliche Öle oder Wachse, Fettsäuren, Fettsäuretriglyceride mit 6 bis 18 Kohlenstoffatomen und Fettalkohole umfaßt.

8. Kosmetische Zusammensetzung nach einem der Ansprüche 1 bis 7, dadurch gekennzeichnet, daß der kosmetische Träger niedrige Monoalkohole oder Polyalkohole mit 1 bis 6 Kohlenstoffatomen, Wasser oder eine Mischung dieser Verbindungen enthält.

9. Kosmetische Zusammensetzung nach einem der Ansprüche 1 bis 8, dadurch gekennzeichnet, daß sie außerdem kosmetische Hilfsstoffe enthält, ausgewählt aus nichtionischen, anionischen, kationischen oder amphoteren Emulgatoren, Verdikkungsmitteln, Hydratationsmitteln, Weichmachern, Konservierungsmitteln, Farbstoffen, Trübungsmitteln, Mitteln zur Regulierung des pH-Werts, Treibmitteln und Parfüms.

10. Verfahren zur Herstellung einer kosmetischen Zusammensetzung zum Schutz der Haut gegen ultraviolette Strahlung der Wellenlängen zwischen 280 und 400 nm, dadurch gekennzeichnet, daß man in einer ersten Stufe 0,01 bis 5 Gew.-%, bezogen auf das Gesamtgewicht der Zusammensetzung, Kupfer (II)-bis(3,5-diisopropylsalicylat) in einer vorher auf 40° bis 90° C erhitzten Fettphass löst, die mindestens einen Bestandteil enthält ausgewählt aus den mineralischen, tierischen und pflanzlichen Ölen oder Wachsen, Fettsäuren, Fettsäuretriglyceriden mit 6 bis 18 Kohlenstoffatomen und den Fettalkoholen, löst, und dann in einer zweiten Stufe mindestens einen kosmetischen Hilfsstoff zugibt, ausgewählt aus den niederen Monoalkoholen oder Polyalkoholen mit 1 bis 6 Kohlenstoffatomen, Wasser oder Mischungen dieser Verbindungen, nichtionischen, anionischen, kationischen oder amphoteren Emulgatoren, Verdickungsmitteln, Hydratationsmitteln, Weichmachern, Konservierungsmitteln, Farbstoffen, Trübungsmitteln, Mitteln zur Regulierung des pH-Wertes, Treibmitteln und Parfüms.

11. Verfahren nach Anspruch 10, dadurch gekennzeichnet, daß man in der Fettphase 0.05 bis 1 Gew. %, bezogen auf das Gesamtgewicht der Zusammensetzung, Kupfer(II)-bis(3,5-diisopropylsalicylat) löst.

12. Verfahren nach Anspruch 10 oder 11, dadurch gekennzeichnet, daß man zu der kosmetischen Zusammensetzung 0.5 bis 20 Gew. %, bezogen auf das Gesamtgewicht der Zusammensetzung, eines oder mehrerer Sonnenfilter zugibt, die ultraviolette Strahlen im Wellanlängenbereich von 280 bis 400 nm absorbieren, ausgewählt aus den Derivaten der p-Aminobenzoesäure, Zimtsäure, Benzylidenkampfer, Salicylsäure, Anthranilsäure, Urocansäure, von Benzophenon, Dibenzoylmethan, Benzotriazol und Benzimidazolsulfonsäure.

13. Verfahren nach einem dar Ansprüche 10 bis 12 zur Herstellung einer kosmetischen Zusammensetzung als Emulsion, in Form einer Creme oder einer Schutzmilch, dadurch gekennzeichnet, daß man unter heftiger Bewegung zu einer vorher auf 75° bis 80° C erhitzten Fettphase, in der man Kupfer (II)-bis(3,5-diisopropylsalicylat) und Emulgatoren gelöst hat, die auf die gleiche Temperatur erhitzte wässerige Phase zugibt, die Bewegung während 10 bis 15 Minuten aufrecht erhält, dann unter abgeschwächter Bewegung abkühlen läßt, und bei etwa 40° C Parfüm und Konservierungsmittel zugibt.

14. Verfahren nach einem der Ansprüche 10 bis 12 zur Herstellung einer kosmetischen Zusammensetzung in Form eines Fettgels, eines Öls, einer Lotion oder eines ölalkoholischen Gels, dadurch gekennzeichnet, daß man die Mischung von Kupfer(II)-bis(3,5-diisopropylsalicylat), der fett- oder ölalkoholischen Phase und der kosmetischen Hilfsstoffe auf 40° bis 50°C erwärmt.
